# EUROPÄISCHE PATENTSCHRIFT

(11) **EP 0 655 926 B1**
(45) Veröffentlichungstag und Bekanntmachung des Hinweises auf die Patenterteilung: **28.01.1998**
(21) Anmeldenummer: 93917719.2
(22) Anmeldetag: 02.08.1993
(51) Int. Cl.: A61K 38/00, C07K 2/00, C07K 4/00, C07K 14/00, C07K 16/00

(54) **NEUE SONDE ZUR TUMORDIAGNOSTIK ODER TUMORTHERAPIE**
NEW TUMOR DIAGNOSING OR THERAPEUTICAL PROBE
NOUVELLE SONDE UTILISEE POUR DIAGNOSTIQUER OU TRAITER DES TUMEURS

(30) Priorität: 03.08.1992 DE 4225569
(43) Veröffentlichungstag der Anmeldung: 07.06.1995
(73) Patentinhaber: MAX-PLANCK-GESELLSCHAFT ZUR FÖRDERUNG DER WISSENSCHAFTEN E.V., 14195 Berlin (DE)
(72) Erfinder: GRUSS, Peter, D-37085 Göttingen (DE); MAULBECKER, Catharina, CH-8044 Zürich (CH)
(74) Vertreter: Böhm, Brigitte, Dipl.-Chem. Dr.
(86) Internationale Anmeldenummer: EP9302051
(87) Internationale Veröffentlichungsnummer: WO9403196

(56) Entgegenhaltungen:
- WO-A-91/09974
- WO-A-92/13949
- CHEMICAL ABSTRACTS, vol. 118, no. 13, 29. März 1993, Columbus, Ohio, US; abstract no. 117821q, & BIOCHEM. SOC. TRANS. Bd. 20, Nr. 1 , 1992 Seite 29S J.M. FEATHERSTONE ET AL. 'ISOLATION OF A GENE FROM XENOPUS BOREALIS CONTAING A PAIRED BOX.'
- MEDLINE AN=92001534 (KARLSRUHE) & MECH. DEV. Bd. 31, Nr. 1 , März 1991 Seiten 29 - 41 OPSTELTEN DJ. ET AL. 'THE MOUSE HOMEOBOX GENE, S8, IS EXPRESSED DURING EMBRYOGENESIS PREDOMINANTLY IN MESENCHYME.'
- PROCEEDINGS OF THE NATIONAL ACADEMY OF SCIENCES OF USA Bd. 89, Nr. 11 , Februar 1992 , WASHINGTON US Seiten 1179 - 1183 G. R. DRESSLER ET AL 'PAX-2 IS A DNA-BINDING PROTEIN EXPRESSED IN EMBRYONIC KIDNEY AND WILMS TUMOR.'
- DATABASE MEDLINE US NATIONAL LIBRARY OF MEDICINE (NLM), BETHESDA, MD, US AN=92120664 & GENOMICS Bd. 11, Nr. 2 , Oktober 1991 Seiten 424 - 434 C. WALTHER ET AL. 'PAX: A MURINE MULTIGENE FAMILY OF PAIRED BOX- CONTAINING GENES.'
- NATURE Bd. 360, Nr. 6399 , 5. November 1992 , LONDON GB Seiten 87 - 89 S. KRAUSS ET AL. 'ZEBRAFISH PAX B IS INVOLVED IN THE FORMATION OF THE MIDBRAIN-HINDBRAIN BOUNDARY.'

## Beschreibung

Die vorliegende Erfindung betrifft ein Verfahren zur Herstellung eines neuen therapeutischen oder diagnostischen Mittels, das als Wirksubstanz mindestens eine Nukleinsäure enthält, und insbesondere zur in vitro Diagnostik von Tumoren geeignet ist.

Proteine, die eine Homöobox enthalten, spielen eine wichtige Rolle bei der Entwicklung von vielzelligem diffenziertem Gewebe. Man nimmt an, daß die transkriptionale Regulation durch die Homöoboxproteine die genaue räumliche und zeitliche Abfolge von Wachstum und Differenzierung in dem sich entwickelnden Embryo koordiniert. Aus der Literatur (sie z. B. K. Kongsuwan, J. M. Adams, Nucleic Acids Res. 17 (1989), 1881-1891; C. Blatt, D. Aberdam, R. Schwartz, L. Sachs, EMBO J. 7 (1988), 4283-4290; C. Blatt, Cancer Cells 2 (1990), 186-189; T. H. Rabbitts Cell 647 (1991), 641-644; A. W. Sasaki, J, Doskow, C. L. Macleod, M. B. Rodgers, L. J. Goudas, M. F. Wilkinson, MOD34 (1991), 155-164) ist bekannt, daß einige Homöobox-enthaltende Gene mit der Onkogenese in Zusammenhang stehen.

Eine Multigenfamilie, die ein gemeinsames konserviertes Sequenzmotiv, die "Paired"-Box aufweist, steht ebenfalls mit der Entwicklungskontrolle und der Gewebespezifität in verschiedenen Organismen im Zusammenhang. Die von der "Paired"-Box kodierte "Paired"-Domäne stellt eine DNA-bindende Domäne dar (J. Treismann, E. Harris, C. Desplan, Genes. Dev. 5 (1991), 594-604; G. Chalepakis, R. Fritsch, H. Fickenscher, O. Deutsch, M. Goulding, P. Gruss, Cell 66 (1991), 873-884) und wurde in verschiedenen Organismen, wie etwa Drosophila, Maus, Schildkröte, Zebrafisch, Nematoden und Menschen identifiziert. Ein Zusammenhang der "Paired"-Domäne mit der Onkogenese war bisher nicht bekannt.

In der medizinischen Forschung werden große Anstrengungen auf die Bereitstellung neuer therapeutischer und diagnostischer Mittel im Zusammenhang mit der Entstehung von Tumoren unternommen. Aufgabe der vorliegenden Erfindung war die Bereitstellung eines neuen Mittels, das insbesondere zur Diagnose und Therapie von Tumoren geeignet ist.

Die erfindungsgemäße Aufgabe wird durch ein Verfahren zur Herstellung eines Mittels zur Tumordiagnostik oder/und Tumortherapie gelöst, welches dadurch gekennzeichnet ist, daß man eine Wirksubstanz, die
(a) mindestens eine Nukleinsäure, die mit einem Pax-Gen hybridisiert,
(b) mindestens ein Pax-Protein, oder/und
(c) mindestens einen Antikörper gegen ein Pax-Protein oder ein Derivat davon enthält,
gegebenenfalls mit pharmazeutisch üblichen Träger-, Hilfs- und Verdünnungsmitteln formuliert.

Überraschenderweise wurde festgestellt, daß Pax-Proteine, d.h. Proteine, welche die "Paired"-Domäne enthalten, die Onkogenese fördern können und somit als neue Gruppe von starken Onkoproteinen klassifiziert werden können, welche die Zellproliferation, das Verankerungs-unabhängige Wachstum und die Angiogenese induzieren. Es wurde festgestellt, daß mit Pax-Genen transformierte Zellen alle klassischen Malignitätsmerkmale, wie z.B. Kontaktinhibierung im "Focusassay", Wachstum in Weichagar und Tumorinduzierung in der Nacktmaus zeigten.

Das nach dem erfindungsgemäße Verfahren hergestellte therapeutische oder diagnostische Mittel kann somit als molekulare Sonde in der Tumordiagnostik eingesetzt werden, da die Verwendung einer Nukleinsäure, die mit einer für ein Pax-Protein kodierenden Nukleotidsequenz hybridisiert, eine qualitative und quantitative, zell- und gewebsspezifische Bestimmung der Expression des jeweiligen Pax-Gens ermöglicht.

Das nach dem erfindungsgemäßen Verfahren hergestellte Mittel ist jedoch auch als Antisense-Nukleinsäure zur spezifischen Hemmung der Expression von Genen, welche die Pax-Sequenz enthalten, und somit auch als therapeutisches Mittel geeignet.

Ein nach dem erfindungsgemäßen Verfahren hergestelltes Mittel muß zum diagnostischen Nachweis oder/und zur therapeutischen Behandlung mindestens eine Nukleinsäure enthalten, welche eine Bindung mit einem Pax-Gen eingeht. Vorzugsweise hybridisiert die erfinungsgemäße Nukleinsäure unter "stringenten Bedingungen" an ein Pax-Gen. Stringente Bedingungen im Sinne der vorliegenden Erfindung sind als solche Bedingungen definiert, die eine selektive und nachweisbare spezifische Bindung der Nukleinsäure an ein bestimmtes oder mehrere Pax-Gene oder Pax-Transkripte ermöglichen. Eine derartige Hybridisierung unter stringenten Bedingungen bedeutet vorzugsweise, daß nach einer Hybridisierung bei 68° C in einer wäßrigen Lösung oder bei 42° C in 50 % Formamid und anschließendem Waschen des Filters bei 65° C in einer wäßrigen Lösung noch eine Bindung der Sonde an das Pax-Gen oder die Pax-RNA nachweisbar ist. Bei Verwendung von kürzeren Nukleinsäuren als Sonden kann es jedoch erforderlich sein, weniger drastische Hybridisierungs- oder/und Waschbedingungen zu verwenden.

Das nach dem erfindungsgemäßen Verfahren hergestellte therapeutische oder diagnostische Mittel enthält vorzugsweise als Wirksubstanz mindestens eine Nukleinsäure, die (a) die für ein Pax-Protein kodierende Nukleotidsequenz, (b) einen Teil davon, (c) eine mit einer Nukleinsäure aus (a) oder/und (b) unter stringenten Bedingungen hybridisierende (siehe oben) Nukleotidsequenz oder (d) eine zu einer Nukleinsäure aus (a), (b) oder/und (c) komplementäre Nukleotidsequenz umfaßt.

Wenn gewünscht wird, daß die erfindungsgemäße Nukleinsäure aus einem konservierten Bereich des Pax-Gens, d. h. aus der für die "Paired"-Domäne kodierenden Nukleotidsequenz stammen soll, verwendet man vorzugsweise eine Nukleinsäure, die (a) eine für die Aminosäuren 1 bis 74 einer "Paired"-Domäne kodierende Nukleotidsequenz, (b) einen Teil davon, (c) eine mit einer Nukleinsäure aus (a) oder/und (b) unter stringenten Bedinungen hybridisierende Nukleotidsequenz oder (d) eine zu einer Nukleinsäure aus (a), (b) oder/und (c) komplementäre Nukleotidsequenz umfaßt.

In einer weiteren bevorzugen Ausführungsform für den obigen Zweck enthält das nach dem erfindungsgemäßen Verfahren hergestellte Mittel mindestens eine Nukeleinsäure, die (a) eine für die Aminosäuren 5 bis 19, 35 bis 41, 68 bis 74, 95 bis 100 oder/und 115 bis 120 einer "Paired"-Domäne kodierende Nukleotidsequenz, (b) einen Teil davon, (c) eine mit einer Nukleinsäure aus (a) oder/und (b) unter stringenten Bedingungen hybridisierende Nukleotidsequenz oder (d) eine zu einer Nukleinsäure aus (a), (b) oder/und (c) komplementäre Nukleotidsequenz umfaßt.

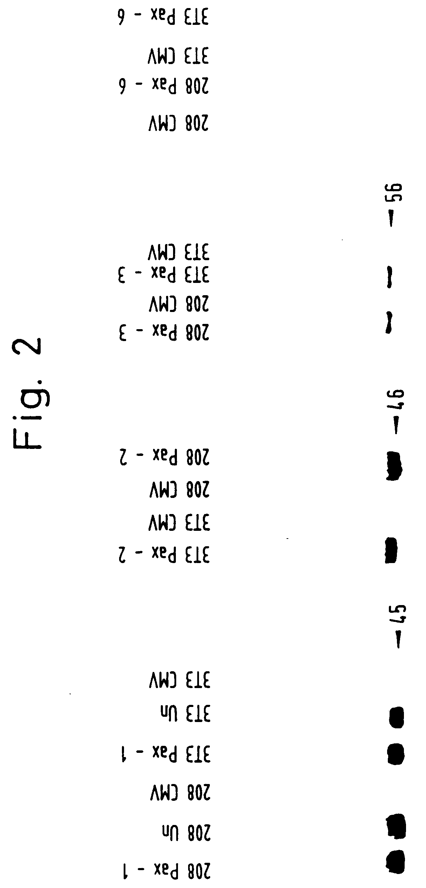

Die voranstehend genannte Nomenklatur der Aminosäuren richtet sich dabei nach der Arbeit Walther et al., Genomics 11 (1991), 424-434, insbesondere Figur 2, die hiermit durch Bezugnahme zu einem Teil der Beschreibung wird.

Wenn es jedoch erforderlich ist, ein einziges Pax-Gen spezifisch nachzuweisen oder zu hemmen, wird man zweckmäßigerweise eine Nukleinsäure mit einer Nukleotidsequenz aus einem nichtkonservierten Bereich des jeweiligen Gens verwenden, d. h. vorzugsweise aus dem nicht für die "Paired"-Domäne kodierenden Bereich.

Das nach dem erfindungsgemäßen Verfahren hergestellte Mittel enthält eine Nukleinsäure, die aus einem beliebigen Pax-Gen stammt. Beispiele für geeignete Pax-Gene sind Pax-1 (Deutsch et al., Cell 53 (1988), 617-625), Pax-2 (Dressler et al., Development 109 (1990), 787-795; Nornes et al., Development 109 (1990, 797-809), Pax-3 (Goulding et al., EMBO J. 10 (1991), 1135-1147), Pax-4, Pax-5 und Pax-6 (Walther et al. (1991), supra), Pax-7 (Jostes et al., MOD 33 (1990), 27-38), Pax-8 ( (Plachov et al., Deveolpment 110 (1990), 643-651), HuP1, HuP2, HuP48 (Burri et al., EMBO J. 8 (1989), 1183-1190), prd, BSH4 und BSH9 (Bopp et al., Cell 47 (1986), 1033-1040) und Pox neuro und Pox meso (Bopp et al., EMBO J. 8. (1989), 3447-3457). Die oben genannten Literaturstellen werden durch Bezugnahme zu einem Teil der Beschreibung. Besonders bevorzugt sind menschliche Pax-Gene.

Die Nukleinsäure in dem nach dem erfindungsgemäßen Verfahren hergestellte Mittel ist - je nach Erfordernis - vorzugsweise eine unmodifizierte oder modifizierte DNA oder RNA. Auch die Länge der Nukleinsäure richtet sich nach dem jeweiligen Anwendungsgebiet.

Bei Verwendung des nach dem erfindungsgemäßen Verfahren hergestellten Mittels als molekulare Sonde in der Tumordiagnostik handelt es sich vorzugsweise um eine DNA-Sonde mit einer Länge von 10 bis 100 Nukleotiden, vorzugsweise 12 bis 50 Nukleotiden. Weiterhin ist es bevorzugt, daß die Nukleinsäure eine radioaktive oder nicht-radioaktive Markierung trägt, die zum Nachweis der Bindung an ein Pax-Gen dient.

Bei Verwendung des nach dem erfindungsgemäßen Verfahren hergestellten Mittels als Antisense-Nukleinsäure zur Hemmung der Genexpression handelt es sich vorzugsweise um eine RNA, die gegebenenfalls modifizierte Basen enthalten kann, um ihre Stabilität im Körper gegenüber einem Abbau durch Ribonukleasen zu erhöhen.

Die Anwendung des nach dem erfindungsgemäßen Verfahren hergestellten Mittels als molekulare Sonde oder/und als therapeutisches Mittel zur Hemmung der Genexpression erfolgt auf eine dem Fachmann auf dem Gebiet der Molekularbiologie bekannte Weise.

Die Erfindung betrifft weiterhin ein nach dem erfindungsgemäßen Verfahren hergestelltes therapeutisches oder diagnostisches Mittel, welches dadurch gekennzeichnet ist, daß es als Wirksubstanz mindestens ein Pax-Protein enthält. Das Pax-Protein ist vorzugsweise aus der Gruppe, bestehend aus Pax-1, Pax-2, Pax-3, Pax-4, Pax-5, Pax-6, Pax-7, Pax-8, HuP1, HuP2, HuP48, prd, BSH4, BSH9, Pox neuro und Pox meso ausgewählt. Die Aminosäuresequenz dieser Proteine ist aus den oben genannten Veröffentlichungen ersichtlich, die in Zusammenhang mit der Nukleinsäuresequenz genannt worden sind. Besonders bevorzugt sind menschliche Pax-Proteine.

Das nach dem erfindungsgemäßen Verfahren hergestellte Mittel wird vorzugsweise in der Tumordiagnostik oder/und Tumortherapie verwendet.

Noch ein weiterer Gegenstand der vorliegenden Erfindung ist ein Verfahren zur in vitro Diagnostik, welches dadurch gekennzeichnet ist, daß es als Wirksubstanz mindestens einen Antikörper gegen ein Pax-Protein enthält. Bevorzugt sind Antikörper, die gegen ein oder mehrere Pax-Proteine aus der Gruppe bestehend aus Pax-1, Pax-2, Pax-3, Pax-4, Pax-5, Pax-6, Pax-7, Pax-8, HuP1, Hup2, HuP48, prd, BSH4, BSH9, Pox neuro und Pox meso gerichtet sind. Besonders bevorzugt sind Antikörper gegen menschliche Pax-Proteine.

Die erfindungsgemäßen Antikörper sind vorzugsweise monoklonale Antikörper, die auf bekannte Weise nach der Köhler-Millstein-Methode durch Immunisierung eines Versuchstieres, vorzugsweise einer Maus, mit dem entsprechenden Pax-Protein oder/und einem Gemisch von Pax-Proteinen, Gewinnung von Antikörper-produzierenden B-Zellen oder Milzzellen aus dem immunisierten Versuchstier und anschließender Fusionierung der Antikörper-produzierenden Zellen mit einer geeigneten Leukämiezelle zur Erzeugung von Hybridomen erhältlich sind. Beispiele für geeignete Antikörper sind Pax-1, Pax-2- und Pax-6-Antikörper (Abb.2).

Die erfindungsgemäßen Antikörper können vorzugsweise in vitro oder/und in vivo als Mittel in der Tumordiagnostik oder/und Tumortherapie verwendet werden. Dabei können die Antikörper auch als Fragmente (z.B. Fab oder F(ab)₂ Fragmente) und gegebenenfalls gekoppelt an eine nachweisbare Gruppe (Enzym, Fluoreszenzmarker, radioaktiver Marker, Kernresonanzmarker etc.) oder an ein Toxin (z.B. Rizin, Diphterietoxin etc.) verwendet werden. Die Herstellung derartiger Antikörper-Derivate erfolgt auf eine dem Fachmann auf dem Gebiet der Immunologie bekannte Weise (z.B. durch kovalente Kopplung über einen bi-funktionellen Linker).

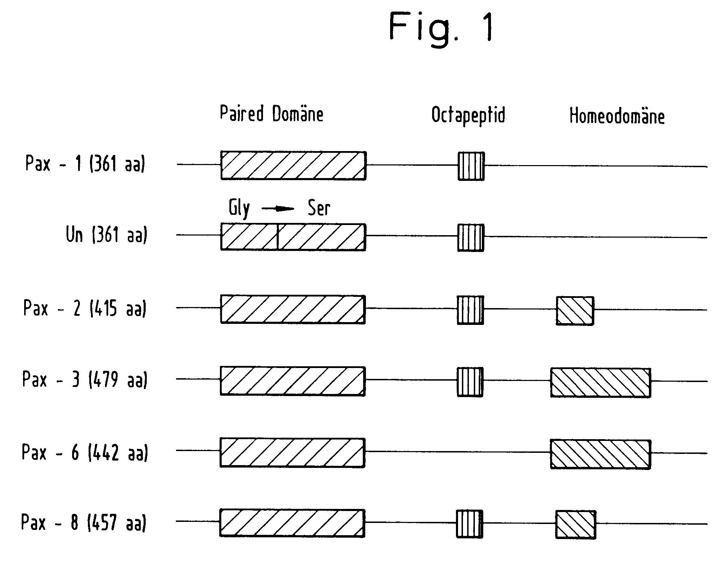

Das folgende Beispiel dient in Verbindung mit Abb. 1 und 2 zur weiteren Veranschaulichung der Erfindung.
**Abb. 1** zeigt den schematischen Aufbau der untersuchten Pax-Proteine Pax-1, Pax-2, Pax-3, Pax-6 und Pax-8 sowie des mutagenisierten Pax-Proteins Un.
**Abb. 2** zeigt Westernblots von Pax-Protein exprimierenden Zellextrakten nach Inkubation mit spezifischen Antikörpern und enzymatischem Nachweis der Antikörper-Protein-Bindung.

### Beispiel

### 1. Untersuchte Pax-Proteine

Es wurde die Wirkung der Proteine Pax-1, Pax-2, Pax-3, Pax-6 und Pax-8 sowie einem mutagenisierten Pax-Protein (Un) untersucht. In Abbildung 1 ist die schematische Struktur dieser Pax-Proteine gezeigt. Die konservierten Domänen innerhalb der Proteine sind als Balken gezeigt, die auch ihre annähernden Positionen innerhalb der offenen Leserahmen angeben. Pax-1 ist das einzige Pax-Protein, von dem ein vollständiges Fehlen der Homöodomäne bekannt ist. Pax-3 und Pax-6 enthalten vollständige Homöodomänen zusätzlich zur "Paired"-Domäne. Beide DNA-Bindungsmotive sind durch mindestens 100 Aminosäuren voneinander getrennt. Die Proteine Pax-2 und Pax-8 enthalten nur 23 Aminosäuren der α-Helix der Homöodomäne. Die Punktmutation von G zu A in dem für das Un-Protein kodierenden Gen ist durch den resultierenden Austausch von Gly nach Ser gekennzeichnet. Die Nukleotid- und Aminosäuresequenzen sind für Pax-1 bei Deutsch et al. (Cell 53 (1988), 617-625), für Pax-2 bei Dressler et al. (Development 109 (1990), 787-795), für Pax-3 bei Goulding et al. (EMBO J. 10 (1991), 1135-1147), für Pax-6 bei Walther und Gruss (Development 113 (1991), 1435-1439) und für Pax-8 bei Plachov et al. (Development 110 (1990), 643-651) offenbart.

### 2. Transformationstest

Die Pax-cDNAs wurden in die multiple Klonierungsstelle des Vektors pCMV5 (Anderson et al., J.Biol.Chem. 264 (1989), 8222-8229) insertiert. Diese Konstrukte wurden zusammen mit pGKneo als selektierbarer Marker (Soriano et al., Cell 64 (1991), 693-702) in 208- und NIH 3T3-Zellen kotransfiziert. Die 208-Zellen wurden in DMEM (Biochrome) und Zusatz von 10 % fötalem Kälberserum (Boehringer Mannheim) kultiviert. Die NIH 3T3-Zellen wurden in DMEM mit 5 % neugeborenem Kälberserum (Boehringer Mannheim) kultiviert. 2 µg des jeweiligen pCMV-Pax-Expressionsplasmids wurden zusammen mit 1 µg pGKneo und 7 µg Träger-DNA auf 70 % konfluenten Einzelzellschichten einer 100 mm Gewebekulturplatte unter Verwendung der Calciumphosphatmethode mit Modifikationen (Weber und Schaffner, Nature 315 (1984), 75-77) transfiziert. Die transfizierten Zellen wurden nach 24 Stunden in drei Gruppen aufgeteilt. Eine Gruppe wurde für 2 bis 4 Wochen abhängig vom Anfang der Focusbildung stehengelassen. Danach wurden die Zellen mit einigen Tropfen Glutaraldehyd (Sigma) und mit 1 % Methylenblau (Sigma) in Wasser angefärbt. Die Gewebekulturplaten wurden mit Wasser gespült und die Foci gezählt.

Ein weiteres Drittel der Zellen wurde entweder in 0,6 %, 0,9 % oder 1,2 % Weichagar ausgesät, wie bei Fidler et al., Anticancer Res. 11 (1991), 17-24 beschrieben ist. Das verbleibende Drittel wurde zur DNA-Aufnahme durch Zugabe von G418 (Gibco) 24 Stunden nach dem Schock für die Zellen ausgewählt. Bei den 208-Zellen wurden 0,4 mg/l und bei den NIH 3T3-Zellen 0,6 mg/ml G418 zugesetzt. Die morphologisch transformierten Foci wurden gepickt und danach kultiviert. Diese Zellisolate wurden durch kontinuierliche Inkubation im Selektionsmedium vermehrt und für die Expressionsanalyse und die Transformationstests verwendet.

### 3. Ergebnisse der Transformationstests

In Tabelle 1 sind die Ergebnisse einer Transformation von 208-Zellen mit Pax-Proteinen gezeigt. Die linke Spalte listet die DNAs auf, die in die Zellen eingeführt wurden. pCMV bedeutet Zellen, die nur das pCMV-Konstrukt als negative Kontrolle enthalten, pSV ist das als positive Kontrolle verwendete T-Antigen (SV40-Virus) Expressionskonstrukt. Die verschiedenen pCMV-Pax-Expressionskonstrukte sind durch den Namen des Pax-Proteins angegeben, das sie exprimieren. Das Wachstum der Zellen wurde in 0,6 %, 0,9 % und 1,2 % Weichagar getestet. Die Zellkolonien wurden 2 bis 3 Wochen nach dem Plattieren gezählt. Die Experimente wurden für jeden Zelltyp mindestens 2 mal durchgeführt. + bedeutet Wachstum in Weichagar. - bedeutet kein Wachstum. +- bedeutet widersprechende Ergebnisse in zwei Experimenten.

Die nächste Spalte listet auf, ob die entsprechenden transformierten Zellen in der Lage waren, bei Vermischen mit normalen 208-Zellen eine Focusbildung zu induzieren oder nicht. Dieses Mischexperiment wurde 2 mal ausgeführt.

Die letzte Spalte zeigt die Anzahl von injizierten Nacktmäusen und die Anzahl von Injektionen, die zu einer Tumorbildung führten. Männliche nackte NMRI Mäuse wurden hierzu subkutan in der Flanke im Alter von 4 Wochen mit 1 bis 5 x 10⁵ transformierten Zellen injiziert. Die Zellen wurden vor der Injektion trypsinisiert und 2 x mit Phosphat-gepufferter Salzlösung gewaschen, um stimulierende Effekte aus dem Serum auszuschließen. Die Tiere wurden für maximal 3 Monate auf einer wöchentlichen Basis auf die Bildung von Tumoren untersucht.

**Tabelle 1**

| Transfizierte DNA | Weichagar-Test | | | Focus-test | Tumorigenizität Anzahl von Injektionen/Anzahl von Tumoren in Nacktmäusen |
|---|---|---|---|---|---|
| | 0,6% | 0,9% | 1,2% | | |
| pCMV | -+ | -- | -- | - | 5/0 |
| pSV | ++ | ++ | ++ | + | 6/6 |
| Pax-1 | ++ | ++ | ++ | + | 6/6 |
| Un | -+ | -- | | - | 6/1 |
| Pax-2 | ++ | ++ | ++ | + | 6/6 |
| Pax-3 | ++ | -+ | ++ | + | 6/6 |
| Pax-6 | ++ | ++ | ++ | + | 6/4 |
| Pax-8 | ++ | ++ | ++ | + | 6/6 |

Die Ergebnisse in Tabelle 1 zeigen das onkogene Potential der Pax-Gene bzw. der "Paired"-Domäne. Bei dem Test, der die unterschiedlichen Pax-Proteine exprimierenden Klone in Weichagar mit unterschiedlichen Konzentrationen zeigt, kann das Wachstum der ansteigenden Weichagarkonzentrationen mit der Wahrscheinlichkeit des Auftretens von Tumoren in Beziehung gesetzt werden. Zellen, die nur den pCMV-Expressionsvektor enthielten, zeigten bei 0,6 % Weichagar und mehr kein Wachstum. Zellen, die das Pax-1-, Pax-2-, Pax-3-, Pax-6- bzw. Pax-8-Protein exprimierten, konnten hingegen bei Konzentrationen bis zu 1,2 % Weichagar wachsen. Ein Wachstum in diesem halbfesten Medium zeigt, daß die Pax-Proteine den Zellen die Fähigkeit zum Verankerungs-unabhängigen Wachstum verleihen. Das mutierte Un-Protein war zu einer vollständigen Transformation dieser Zellen nicht in der Lage, wie durch das Fehlen von Verankerungs-unabhängigem Wachstum bei höheren Weichagar-Konzentrationen ersichtlich ist.

Die durch Injektion von pCMV-Pax-Expressionskonstrukten erzeugten Tumore wurden durch Standard in situ Hybridisierungsprotokolle (Goulding, EMBO J. 10 (1991), 1135-1147) analysiert. Die Zellen in den Pax-Tumoren sind spindelförmig. Die Tumore sind gut mit Gefäßen versorgt und zeigen eine starke extrazelluläre Matrixproduktion. Alle Tumore waren fest und verkapselt.

Weiterhin wurde ein Methylenblau-Test durchgeführt, um die Fähigkeit der Zellen zur Überwindung der Kontakthemmung zu bestimmen. Dieser Test wurde 2 mal in unbehandelten Zellen nach Transfektion durchgeführt. Zellen, welche die transformierende DNA aufgenommen haben, sind in der Lage, nicht transformierte Zellen zu überwachsen, was in dunkel markierten Zellfoci resultiert. In den mit Pax-Genen transformierten Zellen wurden - wie in der positiven Kontrolle mit pSV - die Bildung von Zellfoci beobachtet, während in den mit der negativen Kontrolle pCMV transformierten Zellen und in den nicht transformierten Zellen (208- und NIH 3T3-Zellen) deutlich weniger Foci sichtbar waren.

Die Ergebnisse des Weichagar-Tests stehen mit dem Auftreten von stark gefärbten Foci im Methylenblau-Test bei 208- und NIH 3T3-Zelltransfektionen unter Verwendung von Pax-Proteinen, die funktionelle "Paired"-Domänen enthalten, und der Tumorentstehung in der Nacktmaus im Einklang.

### 4. Immunologischer Nachweis der Pax-Expression in transfizierten Zellen

Aus den nach Punkt 2 transfizierten NIH 3T3- und 208-Zellen wurden nach bekannten Protokollen (Balling et al., Cell 55, (1988), 531-535) Gesamtzellextrakte hergestellt. Nach Bestimmung der Proteinkonzentration wurden jeweils 50 µg der Zellextrakte auf einem 12,5 % SDS-Polyacrylamidgel aufgetrennt und auf eine Immobilon-P-Membran durch halbtrockenen elektrischen Transfer überführt. Die Membran wurde in 5 % Trockenmilchpulver/phosphatgepufferter Salzlösung blockiert und über Nacht mit einer 1:200-Verdünnung der jeweiligen Pax-Antikörper inkubiert und mit der Peroxidase/Diaminobenzidin-Reaktion entwickelt (Balling et al., Supra). Aus Abb. 2 ist ersichtlich, daß Antikörper gegen Pax-1, Pax-2, Pax-3 und Pax-6 eine Reaktion mit den entsprechenden transfizierten Zellen zeigten. Der Pax-2-Antikörper zeigte eine Kreuzreaktion mit Pax-8 und ermöglichte eine Bestätigung der Pax-8-Expression mit den jeweiligen Zellextrakten (nicht dargestellt). Das Molekulargewicht der Pax-Proteine wurde durch Vergleich mit dem Regenbogen-Proteinmarker (Amersham) bestimmt. Das scheinbare Molekulargewicht der Proteine ist in kD angegeben. Sowohl 208- als auch NIH 3T3-Zellextrakte enthielten etwa ähnliche Mengen der jeweiligen Pax-Proteine pro 50 µg Zellextrakt. Dies zeigt an, daß der CMV-Promotor in beiden Zellinien gleichermaßen gut funktioniert. Der Westernblot von Pax-1 zeigt, daß Pax-1 und das mutierte Un-Protein in etwa gleichen Mengen exprimiert werden. Ein weiterer Zellextrakt, der durch Transfektion von Zellen mit einem pSV40 Promotor/Pax-1-Konstrukt hergestellt worden war, enthielt noch höhere Mengen des Pax-Proteins. In allen Fällen zeigten die Westernblots, daß mit pCMV transfizierte Kontrollzellen sehr viel weniger oder keine nachweisbaren Mengen an Protein erzeugten.

## Patentansprüche

1. Verfahren zur Herstellung eines Mittels zur Tumordiagnostik oder/und Tumortherapie,
**dadurch gekennzeichnet,**
daß man eine Wirksubstanz, die
(a) mindestens eine Nukleinsäure, die mit einem Pax-Gen hybridisiert,
(b) mindestens ein Pax-Protein, oder/und
(c) mindestens einen Antikörper gegen ein Pax-Protein oder ein Derivat davon enthält,
gegebenenfalls mit pharmazeutisch üblichen Träger-, Hilfs- und Verdünnungsmitteln formuliert.

2. Verfahren nach Anspruch 1,
**dadurch gekennzeichnet,**
daß das Mittel als Wirksubstanz mindestens eine Nukleinsäure enthält, die mit einem Pax-Gen hybridisiert.

3. Verfahren nach Anspruch 2,
**dadurch gekennzeichnet,**
daß das Mittel als Wirksubstanz mindestens eine Nukleinsäure enthält, die (a) die für ein Pax-Protein kodierende Nukleotidsequenz, (b) einen Teil davon, (c) eine mit einer Nukleinsäure aus (a) oder/und (b) unter stringenten Bedingungen hybridisierende Nukleotidsequenz oder (d) eine zu einer Nukleinsäure aus (a), (b) oder/und (c) komplementäre Nukleotidsequenz umfaßt.

4. Verfahren nach Anspruch 2 oder 3,
**dadurch gekennzeichnet,**
daß das Mittel mindestens eine Nukleinsäure enthält, die (a) eine für die Aminosäuren 1 bis 74 einer "Paired"-Domäne kodierende Nukleotidsequenz, (b) einen Teil davon, (c) eine mit einer Nukleinsäure aus (a) oder/und (b) unter stringenten Bedingungen hybridisierende Nukleotidsequenz oder (d) eine zu einer Nukleinsäure aus (a), (b) oder/und (c) komplementäre Nukleotidsequenz umfaßt.

5. Verfahren nach einem der Ansprüche 2 bis 4,
**dadurch gekennzeichnet,**
daß das Mittel mindestens eine Nukleinsäure enthält, die (a) eine für die Aminosäuren 5 bis 19, 35 bis 41, 68 bis 74, 95 bis 100 oder/und 115 bis 120 einer "Paired"-Domäne kodierende Nukleotidsequenz, (b) einen Teil davon, (c) eine mit einer Nukleinsäure aus (a) oder/und (b) unter stringenten Bedingungen hybridisierende Nukleotidsequenz oder (d) eine zu einer Nukleinsäure aus (a), (b) oder/und (c) komplementäre Nukleotidsequenz umfaßt.

6. Verfahren nach Anspruch 2 oder 3,
**dadurch gekennzeichnet,**
daß das Mittel Nukleotidsequenzen aus dem nicht-konservierten Bereich eines Pax-Gens enthält.

7. Verfahren nach einem der Ansprüche 2 bis 6,
**dadurch gekennzeichnet,**
daß das Mittel mindestens eine Nukleinsäure enthält, die Nukleotidsequenzen eines Pax-Gens, aus der Gruppe der Gene, bestehend aus Pax-1, Pax-2, Pax-3, Pax-4, Pax-5, Pax-6, Pax-7, Pax-8, HuP1, HuP2, HuP48, prd, BSH4, BSH9, Pox neuro und Pox meso, enthält.

8. Verfahren nach einem der Ansprüche 2 bis 7,
**dadurch gekennzeichnet,**
daß die Nukleinsäure eine DNA ist.

9. Verfahren nach einem der Ansprüche 2 bis 7,
**dadurch gekennzeichnet,**
daß die Nukleinsäure eine gegebenenfalls modifizierte RNA ist.

10. Verfahren nach einem der Ansprüche 2 bis 9 als molekulare Sonde in der Tumordiagnostik.

11. Verfahren nach einem der Ansprüche 2 bis 9 als Antisense-Nukleinsäure zur Hemmung der Genexpression.

12. Verfahren nach Anspruch 1,
**dadurch gekennzeichnet**,
daß das Mittel als Wirksubstanz mindestens ein Pax-Protein enthält.

13. Verfahren nach Anspruch 12,
**dadurch gekennzeichnet,**
daß das Mittel mindestens ein Pax-Protein aus der Gruppe, bestehend aus Pax-1, Pax-2, Pax-3, Pax-4, Pax-5, Pax-6, Pax-7, Pax-8, HuP1, HuP2, Hup48, prd, BSH9, Pox neuro und Pox meso, enthält.

14. Verfahren nach Anspruch 1,
**dadurch gekennzeichnet**,
daß das Mittel als Wirksubstanz mindestens einen Antikörper gegen ein Pax-Protein oder ein Derivat davon enthält.

15. Verfahren nach Anspruch 14,
**dadurch gekennzeichnet**,
daß der Antikörper gegen ein oder mehrere Pax-Proteine aus der Gruppe, bestehend aus Pax-1, Pax-2, Pax-3, Pax-4, Pax-5, Pax-6, Pax-7, Pax-8, HuP1, HuP2, Hup48, prd, BSH9, Pox neuro und Pox meso gerichtet ist.

16. Verfahren zur in vitro Diagnostik von Tumoren,
dadurch gekennzeichnet,
daß man eine Wirksubstanz, die
(a) mindestens eine Nukleinsäure, die mit einem Pax-Gen hybridisiert,
(b) mindestens ein Pax-Protein, oder/und
(c) mindestens einen Antikörper gegen ein Pax-Protein oder ein Derivat davon enthält,
verwendet.

## Claims

1. Process for the production of an agent for tumour diagnostics or/and tumour therapy,
**wherein**
an active substance which contains
(a) at least one nucleic acid which hybridizes with a Pax gene,
(b) at least one Pax protein or/and
(c) at least one antibody against a Pax protein or a derivative thereof
is formulated, if desired with common pharmaceutical carrier substances, auxiliary substances and diluents.

2. Process as claimed in claim 1,
**wherein**
the agent contains at least one nucleic acid which hybridizes with a Pax gene as the active substance.

3. Process as claimed in claim 2,
**wherein**
the agent contains as the active substance at least one nucleic acid which comprises (a) a nucleotide sequence coding for a Pax protein, (b) a part thereof, (c) a nucleotide sequence hybridizing under stringent conditions with a nucleic acid from (a) or/and (b) or (d) a nucleotide sequence complementary to a nucleic acid from (a), (b) or/and (c).

4. Process as claimed in claim 2 or 3,
**wherein**
the agent contains at least one nucleic acid which comprises (a) a nucleotide sequence coding for the amino acids 1 to 74 of a "paired" domain, (b) a part thereof, (c) a nucleotide sequence hybridizing under stringent conditions with a nucleic acid from (a) or/and (b) or (d) a nucleotide sequence complementary to a nucleic acid from (a), (b) or/and (c).

5. Process as claimed in one of the claims 2 to 4,
**wherein**
the agent contains at least one nucleic acid which comprises (a) a nucleotide sequence coding for the amino acids 5 to 19, 35 to 41, 68 to 74, 95 to 100 or/and 115 to 120 of a "paired" domain, (b) a part thereof, (c) a nucleotide sequence hybridizing under stringent conditions with a nucleic acid from (a) or/and (b) or (d) a nucleotide sequence complementary to a nucleic acid from (a), (b) or/and (c).

6. Process as claimed in claim 2 or 3,
**wherein**
the agent contains nucleotide sequences from the non-conserved region of a Pax gene.

7. Process as claimed in one of the claims 2 to 6,
**wherein**
the agent contains at least one nucleic acid which contains nucleotide sequences from a Pax gene from the group of genes comprising Pax-1, Pax-2, Pax-3, Pax-4, Pax-5, Pax-6, Pax-7, Pax-8, HuP1, HuP2, HuP48, prd, BSH4, BSH9, Pox neuro and Pox meso.

8. Process as claimed in one of the claims 2 to 7,
**wherein**
the nucleic acid is a DNA.

9. Process as claimed in one of the claims 2 to 7,
**wherein**
the nucleic acid is a RNA which is modified if desired.

10. Process as claimed in one of the claims 2 to 9 as a molecular probe in tumour diagnostics.

11. Process as claimed in one of the claims 2 to 9 as an antisense nucleic acid for the inhibition of gene expression.

12. Process as claimed in claim 1,
**wherein**
the agent contains at least one Pax protein as the active substance.

13. Process as claimed in claim 12,
**wherein**
the agent contains at least one Pax protein from the group comprising Pax-1, Pax-2, Pax-3, Pax-4, Pax-5, Pax-6, Pax-7, Pax-8, HuP1, HuP2, Hup48, prd, BSH9, Pox neuro and Pox meso.

14. Process as claimed in claim 1,
**wherein**
the agent contains as the active substance at least one antibody against a Pax protein or a derivative thereof.

15. Process as claimed in claim 14,
**wherein**
the antibody is directed against one or several Pax proteins from the group comprising Pax-1, Pax-2, Pax-3, Pax-4, Pax-5, Pax-6, Pax-7, Pax-8, HuP1, HuP2, Hup48, prd, BSH9, Pox neuro and Pox meso.

16. Process for the in vitro diagnostics of tumours,
**wherein**
an active substance is used which contains
(a) at least one nucleic acid which hybridizes with a Pax gene
(b) at least one Pax protein or/and
(c) at least one antibody against a Pax protein or
a derivative thereof.

## Revendications

1. Procédé de préparation d'un agent pour le diagnostic des tumeurs et/ou la thérapie des tumeurs, caractérisé en ce que l'on formule, éventuellement avec des supports, adjuvants et diluants courants du point de vue pharmaceutique, une substance active qui contient
(a) au moins un acide nucléique qui s'hybride avec un gène Pax,
(b) au moins une protéine Pax et/ou
(c) au moins un anticorps contre une protéine Pax ou un dérivé de celui-ci.

2. Procédé selon la revendication 1 caractérisé en ce que l'agent contient comme substance active au moins un acide nucléique qui s'hybride avec un gène Pax.

3. Procédé selon la revendication 2 caractérisé en ce que l'agent contient comme substance active au moins un acide nucléique qui comprend (a) la séquence nucléotidique codant une protéine Pax, (b) une partie de celle-ci, (c) une séquence nucléotidique qui s'hybride dans des conditions strictes avec un acide nucléique de (a) et/ou (b) ou (d) une séquence nucléotidique complémentaire d'un acide nucléique de (a), (b) et/ou (c).

4. Procédé selon la revendication 2 ou 3 caractérisé en ce que l'agent contient au moins un acide nucléique qui comprend (a) une séquence nucléotidique codant les acides aminés 1 à 74 d'un domaine "paired", (b) une partie de celle-ci, (c) une séquence nucléotidique qui s'hybride dans des conditions strictes avec un acide nucléique de (a) et/ou (b) ou (d) une séquence nucléotidique complémentaire d'un acide nucléique de (a), (b) et/ou (c).

5. Procédé selon l'une des revendications 2 à 4 caractérisé en ce que l'agent contient au moins un acide nucléique qui comprend (a) une séquence nucléotidique codant les acides aminés 5 à 19, 35 à 41, 68 à 74, 95 à 100 et/ou 115 à 120 d'un domaine "paired", (b) une partie de celle-ci, (c) une séquence nucléotidique qui s'hybride dans des conditions strictes avec un acide nucléique de (a) et/ou (b) ou (d) une séquence nucléotidique complémentaire d'un acide nucléique de (a), (b) et/ou (c).

6. Procédé selon la revendication 2 ou 3 caractérisé en ce que l'agent contient des séquences nucléotidiques du domaine non conservé d'un gène Pax.

7. Procédé selon l'une des revendications 2 à 6 caractérisé en ce que l'agent contient au moins un acide nucléique qui contient des séquences nucléotidiques d'un gène Pax du groupe des gènes consistant en Pax-1, Pax-2, Pax-3, Pax-4, Pax-5, Pax-6, Pax-7, Pax-8, HuP1, HuP2, HuP48, prd, BSH4, BSH9, Pox neuro et Pox meso.

8. Procédé selon l'une des revendications 2 à 7 caractérisé en ce que l'acide nucléique est un ADN.

9. Procédé selon l'une des revendications 2 à 7 caractérisé en ce que l'acide nucléique est un ARN éventuellement modifié.

10. Procédé selon l'une des revendications 2 à 9 comme sonde moléculaire dans le diagnostic des tumeurs.

11. Procédé selon l'une des revendications 2 à 9 comme acide nucléique antisens pour l'inhibition de l'expression génique.

12. Procédé selon la revendication 1 caractérisé en ce que l'agent contient au moins une protéine Pax comme substance active.

13. Procédé selon la revendication 12 caractérisé en ce que l'agent contient au moins une protéine Pax du groupe consistant en Pax-1, Pax-2, Pax-3, Pax-4, Pax-5, Pax-6, Pax-7, Pax-8, HuP1, HuP2, Hup48, prd, BSH9, Pox neuro et Pox meso.

14. Procédé selon la revendication 1 caractérisé en ce que l'agent contient comme substance active au moins un anticorps contre une protéine Pax ou un dérivé de celui-ci.

15. Procédé selon la revendication 14 caractérisé en ce que l'anticorps est dirigé contre une ou plusieurs protéines Pax du groupe consistant en Pax-1, Pax-2, Pax-3, Pax-4, Pax-5, Pax-6, Pax-7, Pax-8, HuP1, HuP2, Hup48, prd, BSH9, Pox neuro et Pox meso.

16. Procédé pour le diagnostic in vitro de tumeurs caractérisé en ce que l'on utilise une substance active qui contient
(a) au moins un acide nucléique qui s'hybride avec un gène Pax,
(b) au moins une protéine Pax et/ou
(c) au moins un anticorps contre une protéine Pax ou un dérivé de celui-ci.
